# EUROPEAN PATENT APPLICATION

(11) **EP 4 825 952 A1**
(43) Date of publication of application: **21.10.2026**
(21) Application number: 23961329.2
(22) Date of filing: 11.12.2023
(51) Int. Cl.: C12M 1/00

(54) **BIOLOGICAL SAMPLE PROCESSING DEVICE**

(71) Applicant: Hitachi High-Tech Corporation, Minato-ku Tokyo 105-6409 (JP)
(72) Inventor: MITSUYAMA Toshifumi, Tokyo 100-8280 (JP); FUJIOKA Michiru, Tokyo 105-6409 (JP)
(74) Representative: MERH-IP Matias Erny Reichl Hoffmann Patentanwälte PartG mbB
(86) International application number: PCT/JP2023/044171
(87) International publication number: WO 2025/126259

(57) **Abstract**

An object of the present invention is to provide a biological sample processing device capable of recovering an eluate, which is a final product, at a high recovery rate. In the biological sample processing device according to the present invention, a pipette performs an aspiration operation in a state in which a tip end of the pipette is in contact with a magnetic beads, and after aspiration of a biological sample, the pipette waits for a period of time sufficient for the magnetic beads in the pipette to be discharged to the outside of the pipette by a magnet.

## Description

### Technical Field

The present invention relates to a technique for processing a biological sample containing a nucleic acid and a magnetic particle.

### Background Art

In recent years, information obtained through a nucleic acid analysis, such as a cancer genome profiling test using a next-generation sequencing (NGS) system, has been utilized in various fields including healthcare, clinical testing, the pharmaceutical industry, the food industry, and the like. In such a nucleic acid analysis, it is essential to extract a nucleic acid from biological samples of various kinds, such as blood, tissues, and cultured cells, as a pretreatment step.

As a method of extracting a nucleic acid, a method based on the property of the nucleic acid to bind to silica in the presence of a chaotropic agent, and a method based on the property of the nucleic acid to bind to silica in the presence of an organic solvent, are commonly used instead of a method of using a harmful organic solvent such as phenol and chloroform. Utilizing these methods, there have been reported a method of extracting a nucleic acid using a nucleic acid capture chip incorporating a silica-containing solid phase as a nucleic acid capture carrier, and a method of using magnetic beads (nucleic acid capture carriers) having surfaces coated with silica. These methods include a binding step in which a nucleic acid binds to the nucleic acid capture carrier, and an elution step in which the nucleic acid is eluted from the nucleic acid capture carrier using an elution liquid.

In the method of using magnetic beads, after the elution step, the magnetic beads are recovered from the eluate using a magnet. One example thereof is a method in which the eluate containing magnetic beads is aspirated into a dispensing tip, the magnetic beads are retained within the dispensing tip using a magnet, and only the eluate is discharged from the dispensing tip. Another example is a method in which magnetic beads are recovered from an eluate containing the magnetic beads by inserting a rod-shaped magnet covered by a cover into the eluate.

Patent Literature 1 describes, as a method for efficiently collecting magnetic beads, a method in which the magnetic beads are first collected on a wall surface of a container and then recovered using a magnetic rod. Patent Literature 2 describes an example in which a nucleic acid is extracted from a large-volume (2 mL) sample by using a thick magnetic comb and a thin magnetic comb in accordance with a procedure shown in FIG. 23. Patent Literature 3 describes a technique in which collection of a magnetic material from a liquid containing the magnetic material is carried out not on the container side where a sample is contained, but by a dispensing apparatus that performs aspiration and discharge of the liquid containing the magnetic material.

### Citation List

### Patent Literature

[Patent Literature 1] US 6,020,211
[Patent Literature 2] US 2022/0176369 A1
[Patent Literature 3] Japanese Patent No. 3115501

### Summary of Invention

### Technical Problem

As one example of a biological sample processing device, a nucleic acid extraction device is assumed. It is assumed that this device is also used, particularly in a test utilizing a liquid biological sample, in a case where extraction of a gene from a large volume of sample (from several mL to several tens of mL) is required. Furthermore, taking the subsequent testing step into consideration, it is desirable that the volume of the sample liquid after the extraction be concentrated to a small volume (several tens of µL).

When the amount of the sample is large, the volume of reagent(s) used, including magnetic beads, also increases accordingly. The magnetic beads capture a nucleic acid from the sample, and then are subjected to a washing step, are placed in an elution liquid, and are removed after the elution of the nucleic acid. When the magnetic beads are to be removed from the eluate using a magnetic rod, the magnetic rod is placed in the elution liquid so that a magnet at a tip end of the magnetic rod attracts the magnetic beads, and the magnetic rod is then pulled out in this state. At this time, the magnetic beads are recovered with the eluate adsorbed thereon, which results in a large-volume loss of the recovered eluate.

In order to address this problem, for example, there is a method in which the elution process of the nucleic acid extraction is carried out twice, as shown in FIG. 23 of Patent Literature 2. This method involves, for example, first eluting a nucleic acid in a medium-volume (several hundreds of µL) elution liquid, then allowing the nucleic acid to bind to a small amount of magnetic beads, and then eluting the nucleic acid again in a small-volume (several hundreds of µL) elution liquid. In this method, the nucleic acid and the magnetic beads are subjected to the adsorption (magnetic collection) and the elution twice. Thus, the method may cause a loss during the intermediate steps, which may result in a decrease in a recovery rate of the nucleic acid. Furthermore, the method also requires a longer processing time.

The present invention has been made in view of the problems described above, and it is an object of the present invention to provide a biological sample processing device capable of recovering an eluate, which is a final product, at a high recovery rate.

### Solution to Problem

In a biological sample processing device according to the present invention, a pipette performs an aspiration operation in a state in which a tip end of the pipette is in contact with a magnetic beads, and after aspiration of a biological sample, the pipette waits for a period of time sufficient for the magnetic beads in the pipette to be discharged to the outside of the pipette by a magnet.

### Advantageous Effects of Invention

According to the present invention, it is possible to provide a biological sample processing device capable of recovering an eluate, which is a final product, at a high recovery rate. As a result, the recovery rate of the eluted final product (such as a nucleic acid) is also improved, and, for example, improvement in accuracy of a test after the processing can be expected.

### Brief Description of Drawings

[FIG. 1] FIG. 1 is a perspective view of a biological sample processing device 1 according to a first embodiment.
[FIG. 2] FIG. 2 is a diagram showing details of each element of the biological sample processing device 1.
[FIG. 3] FIG. 3 is a diagram illustrating a stirring operation.
[FIG. 4] FIG. 4 is a diagram illustrating a magnetic collection operation.
[FIG. 5] FIG. 5 is a flowchart of an eluate recovery operation after an elution step.
[FIG. 6] FIG. 6 is a diagram illustrating an eluate aspiration operation in the eluate recovery operation.
[FIG. 7] FIG. 7 is a diagram showing changes in a pressure measured by a pressure sensor provided in a pipette during recovery of an eluate.
[FIG. 8] FIG. 8 is a diagram illustrating how loss of an eluate to be recovered occurs in a conventional biological sample processing device.
[FIG. 9] FIG. 9 is a side view illustrating set positions used in an eluate recovery operation according to a second embodiment.
[FIG. 10] FIG. 10 is a flowchart of the eluate recovery operation after an elution step according to the second embodiment.
[FIG. 11] FIG. 11 is a diagram illustrating an eluate aspiration operation in the eluate recovery operation.
[FIG. 12] FIG. 12 is a diagram showing a pipette unit 200 of a biological sample processing device 1 according to a third embodiment.

### Description of Embodiments

### First Embodiment

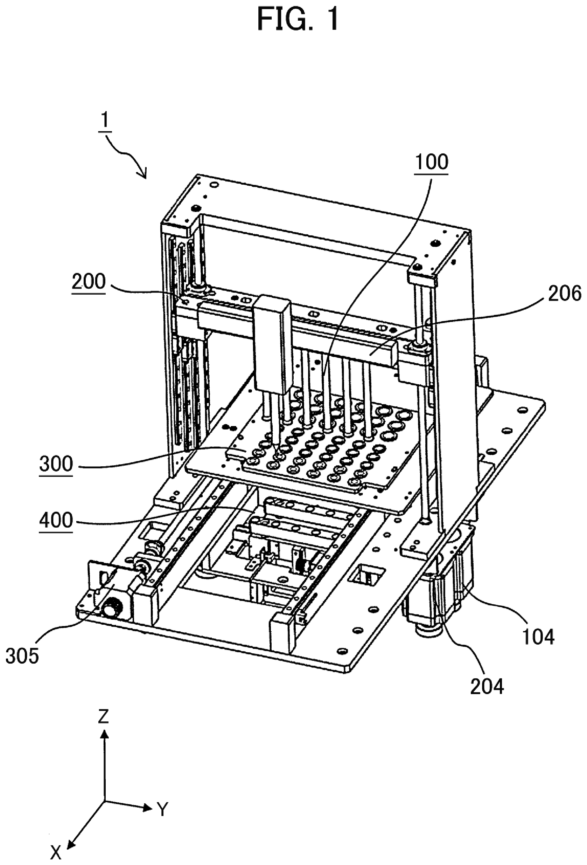

FIG. 1 is a perspective view of a biological sample processing device 1 according to a first embodiment of the present invention. In the first embodiment, the present invention is applied to a nucleic acid extraction device, which is a biological sample processing device. The biological sample processing device 1 is a device configured to extract a nucleic acid from a sample containing the nucleic acid and magnetic particles. The biological sample processing device 1 includes a magnetic rod unit 100, a pipette unit 200, a container conveying unit 300, and a bottom magnet unit 400. A horizontal movement motor 206, a pipette upward and downward movement motor 204, and a container movement motor 305 will be described later.

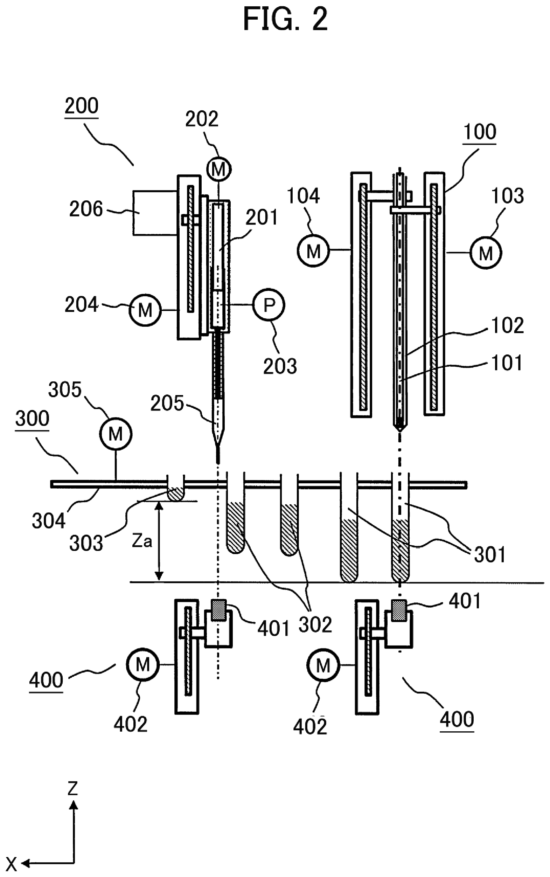

FIG. 2 is a diagram showing details of each element of the biological sample processing device 1. The magnetic rod unit 100 is a unit configured to move a magnetic rod 101 and a magnetic rod cover 102 upward and downward in a vertical direction (Z-axis direction in the drawing) in order to stir a sample in a container and recover magnetic beads contained in the sample. A tip end or the entirety of the magnetic rod 101 is comprised of a neodymium magnet, and a magnetic force acts in the upward and downward direction (Z-axis direction in the drawing) so that collection of magnetic beads in a liquid is enabled. The magnetic rod cover 102 has a hollow shape such that the magnetic rod 101 can be inserted therein, and is made of a resin (such as polypropylene). An inner diameter of the magnetic rod cover 102 is approximately equal to an outer diameter of the magnetic rod 101.

The magnetic rod unit 100 includes the magnetic rod 101, the magnetic rod cover 102, a magnetic rod drive motor 103 for driving them, and a magnetic rod cover drive motor 104. By providing a drive motor for each of the magnetic rod 101 and the magnetic rod cover 102 in this manner, the magnetic rod 101 and the magnetic rod cover 102 can be independently driven.

The pipette unit 200 is a unit including a pipette for aspirating and discharging a sample. This unit includes a plunger 201 for generating an aspiration pressure and a plunger drive motor 202 for operating the plunger 201. The plunger drive motor 202 is controlled by a controller (not shown), and its speed can be arbitrarily set and changed, which allows an aspiration speed of the pipette to be changed according to the situation.

The pipette unit 200 also includes a pressure sensor 203 for measuring the aspiration pressure of the pipette. The pressure sensor 203 is capable of measuring the pressure inside the pipette, and thus it is possible to measure the aspiration pressure of the pipette in real time during an aspiration operation. Furthermore, data of the measured pressure is sent to a computing means (not shown) and can be used for controlling each motor. Moreover, the pipette unit 200 includes the pipette upward and downward movement motor 204 for moving upward and downward the pipette in the Z direction in the drawing.

In the present embodiment, the pipette unit 200 includes one pipette, and this pipette is movable in the Y direction by the horizontal movement motor 206, and thus it can access all containers. The number of pipettes is arbitrary, and a plurality of pipettes may be provided.

The container conveying unit 300 is a unit for moving a container containing a sample in a horizontal direction (X-axis direction) according to each process. The container conveying unit 300 includes a container holder 304 for supporting the container, and the container movement motor 305 for moving the container holder 304 and each container.

The biological sample processing device 1 includes three types of containers used for processing: a large container 301, a medium container 302, and a small container 303, and these containers are arranged at predetermined intervals in the X and Y directions in the drawing.

The bottom magnet unit 400 is a unit for bringing a magnet into contact with and separating the magnet from a bottom of the container containing the sample. In the present embodiment, two bottom magnet units 400 are provided. Each of the bottom magnet unit 400 includes, for example: a bottom magnet 401 comprised of a neodymium magnet; and an actuator 402 for moving upward and downward the bottom magnet 401 in the vertical direction (Z-axis direction in the drawing). When the bottom magnet 401 approaches the bottom surface of the container, magnetic beads can be magnetically collected on the bottom surface of the container.

Among the two bottom magnet units 400, one is disposed directly below the magnetic rod 101 and the magnetic rod cover 102 that are provided in the magnetic rod unit 100, and can be used when the magnetic rod unit 100 processes a sample. The other is disposed directly below a pipette tip 205 of the pipette unit 200, and can be used when the pipette unit 200 processes the sample.

In the biological sample processing device 1, a stirring operation for stirring a liquid in the container, a magnetic collection operation for collecting and recovering magnetic beads present in the liquid using the magnetic rod 101, and a release operation for releasing the recovered magnetic beads back into the liquid are performed by the upward and downward movement of the magnetic rod unit 100.

Processing by the magnetic rod unit 100 is carried out by horizontally moving (in the X direction in the drawing) the container to be subjected to the processing to directly below the magnetic rod 101 and the magnetic rod cover 102, and moving upward and downward the magnetic rod 101 or the magnetic rod cover 102.

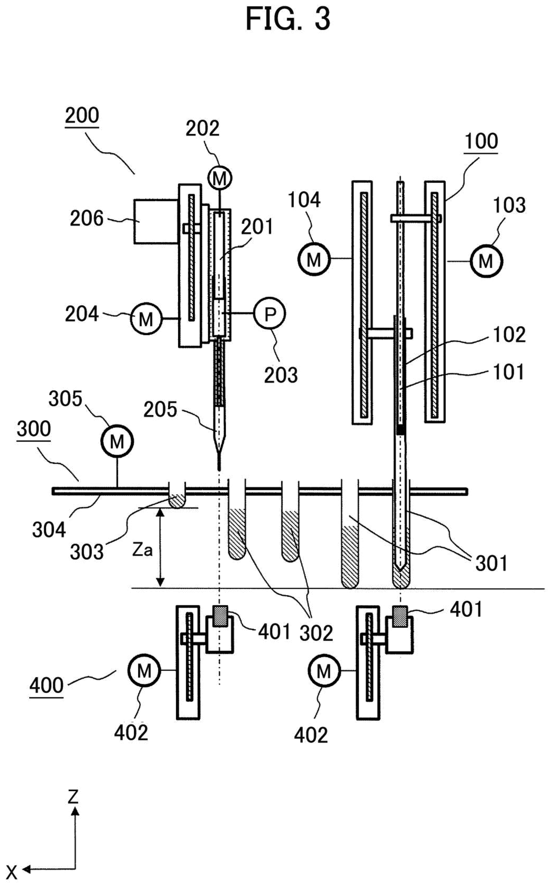

FIG. 3 is a diagram illustrating the stirring operation. The stirring operation is an operation of stirring a solution in a container to be subjected to the stirring, and is carried out by inserting only the magnetic rod cover 102 into the container and moving upward and downward the magnetic rod cover 102, as shown in FIG. 3.

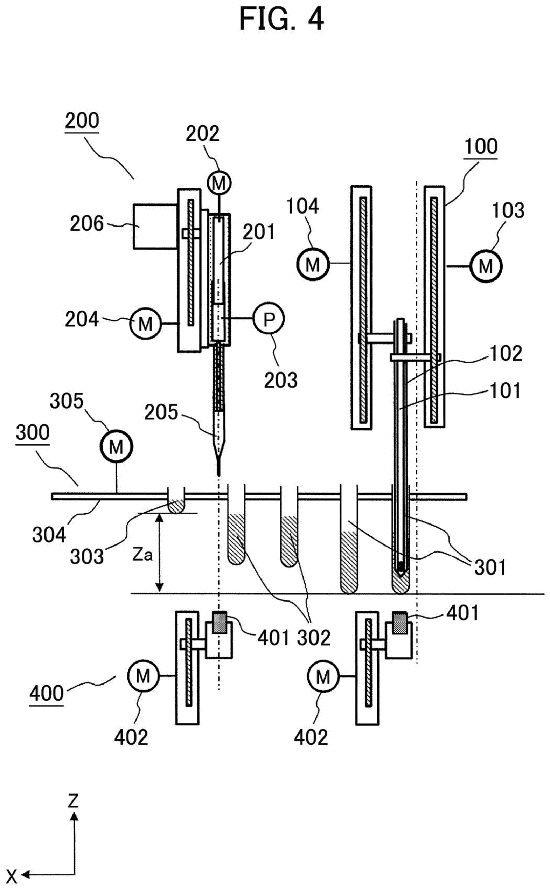

FIG. 4 is a diagram illustrating the magnetic collection operation. The magnetic collection operation is an operation of collecting and recovering magnetic beads by the magnetic rod 101. At this time, as shown in FIG. 4, the magnetic rod 101 is inserted into the magnetic rod cover 102 so that the solution and the magnetic beads do not directly contact the magnetic rod 101, and the magnetic rod cover 102 and the magnetic rod 101 are moved upward and downward at the same time in the liquid within the container, to stir the sample solution and capture the magnetic beads. More preferably, by moving upward and downward the magnetic rod 101 while bringing the bottom magnet 401 close to the wall surface (bottom) of the container, the magnetic beads can be captured at two locations, namely the container wall surface (bottom) and the magnetic rod 101, and thus the capture efficiency can be improved.

The release operation is an operation of releasing the magnetic beads captured by the magnetic rod 101 into the solution. This operation is performed when the magnetic beads are transferred between containers, especially when the magnetic beads magnetically collected in a source container are placed into a destination container. In a state in which the magnetic beads are magnetically collected at the magnetic rod 101, the magnetic rod 101 and the magnetic rod cover 102 are placed into the solution, and thereafter, only the magnetic rod 101 is pulled out. As a result, the magnetic force no longer acts on the tip end of the magnetic rod cover 102, and the magnetically collected magnetic beads are released into the solution. In this state, the magnetic rod cover 102 may also be moved upward and downward in the solution to shake off the magnetic beads adhering to the tip end thereof. Alternatively, the magnetic beads may be separated from the magnetic rod cover 102 by using the bottom magnet 401 to attract the magnetic beads toward the bottom of the container.

The biological sample processing device 1 performs the following three steps (1) to (3) in order to extract the nucleic acid from the sample.
(1) Binding Step: This is a process for binding a nucleic acid present in a sample to magnetic beads. Magnetic beads are added to a large-volume liquid specimen sample (whole blood, plasma, serum, or the like) in the large container 301. At this time, the amount of the magnetic beads to be added is adjusted according to the amount of the liquid specimen sample. In general, when the amount of the sample is large, the amount of the magnetic beads to be added is also large. Furthermore, a reagent that promotes binding between the nucleic acid to be captured and the magnetic beads is added as necessary. Then, the sample solution is stirred using the magnetic rod cover 102 so that the magnetic beads and biological substances bind together. After sufficient binding between the magnetic beads and the nucleic acid, the magnetic beads in the liquid are magnetically collected and recovered.
(2) Washing Step: This is a step of washing the magnetic beads having a nucleic acid bound thereto and removing nonspecifically bound substances from the magnetic beads. This step is carried out by placing the magnetic beads bound to the nucleic acid into the medium container 302 which contains a washing reagent, and stirring the mixture. The washing reagent is not particularly limited, as long as it is capable of removing the reagent added in the Step 1 and contaminants from the nucleic acid capture carrier while maintaining the binding of the nucleic acid to the nucleic acid capture carrier, for example. For example, an organic compound such as a lower alcohol and a low molecular-weight ketone may be used. Alternatively, ethanol, isopropanol, or the like may be used as the washing reagent, and it is particularly preferable to use ethanol having a concentration of 70% or higher. After washing the magnetic rod cover 102 and the magnetic beads, alcohol components and the like are dried so as to prevent the washing liquid from being mixed into the nucleic acid elution liquid to be used in the next step. Means for drying is not particularly limited, but alcohol components evaporate by holding the magnetic rod cover 102, into which the magnetic rod 101 has been inserted, above the washing container.
(3) Elution Step: This is a step of eluting the nucleic acid that has been bound to and captured by the magnetic beads and subsequently washed, from the sample into the elution liquid. Since the resulting eluate is to be sent to a subsequent testing step, it is generally desirable that the volume of the elution liquid be small, and a volume of approximately from 30 to 50 µL is assumed here. After completion of the washing step, the magnetically collected magnetic beads are released into the small container 303 and immersed in a container containing the nucleic acid elution liquid, followed by stirring in that state. As a result, the nucleic acid is eluted into the nucleic acid elution liquid.

The sample after completion of the elution contains the nucleic acid to be analyzed in the liquid, and this eluate is recovered. At this time, unnecessary magnetic beads remain immersed in the eluate, and therefore these magnetic beads need to be removed. The magnetic beads contained in the solution are present in a large amount because binding has been performed in a large-volume liquid. An operation for recovering a small amount of eluate containing such a large amount of magnetic beads will be described below. Recovery of the eluate is carried out using the pipette unit 200.

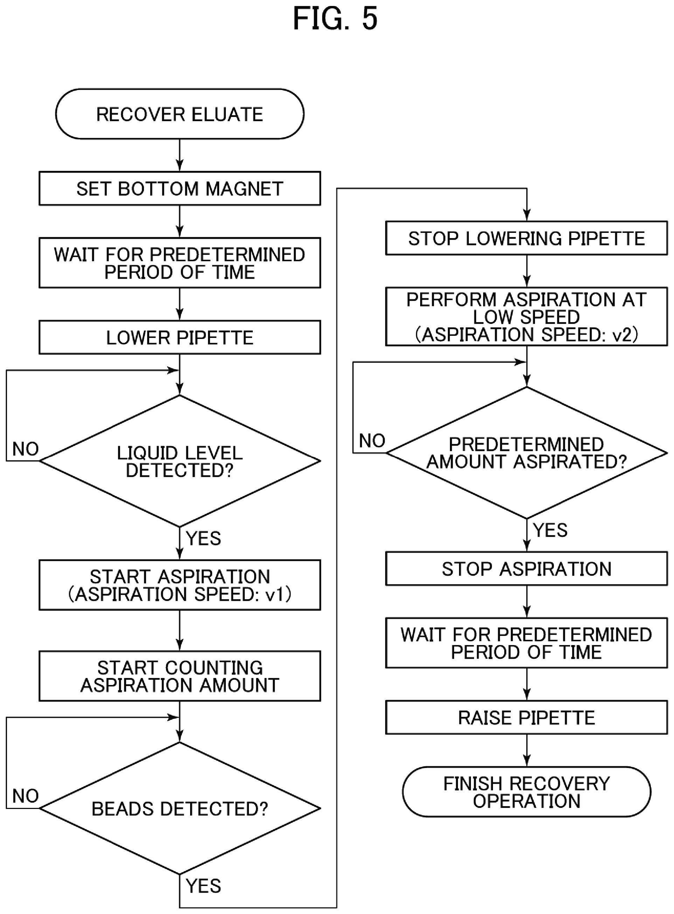

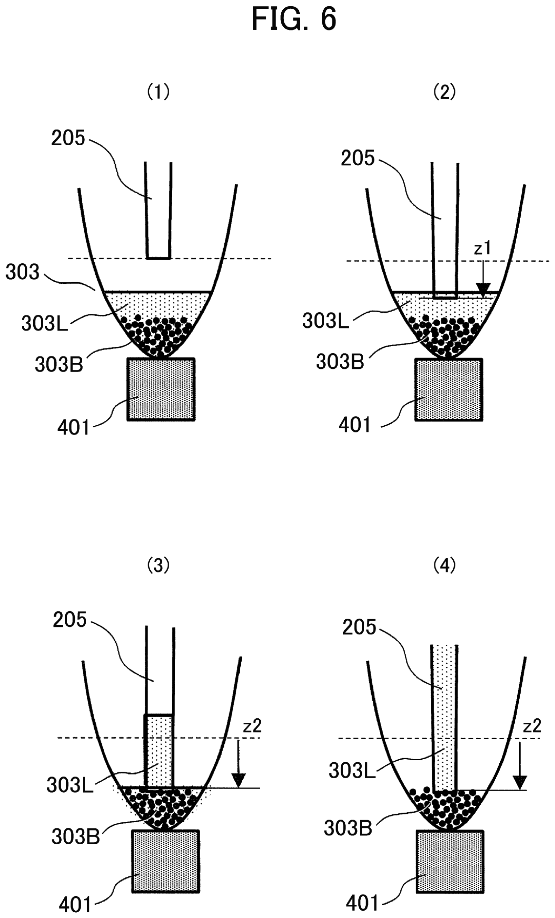

FIG. 5 is a flowchart of an eluate recovery operation after an elution step. FIG. 6 is a diagram illustrating an eluate aspiration operation in the eluate recovery operation. In order to recover the eluate adsorbed onto the magnetic beads, the biological sample processing device 1 performs the aspiration operation in a state in which the tip end of the pipette tip 205 is in contact with the surface of magnetic beads 303B that have been collected and deposited at the bottom of the small container 303 by the bottom magnet 401. Hereinafter, the eluate recovery operation will be described with reference to these drawings.

FIG. 6(1): Immediately after the completion of the elution step, the magnetic beads are floating and dispersed in the eluate. Therefore, before starting aspiration of the eluate, the bottom magnet 401 is brought into contact with the bottom of the small container 303, and the device waits for a predetermined period of time. As a result, the magnetic beads 303B are magnetically collected at the bottom of the small container 303 (from setting of the bottom magnet to waiting for a predetermined period of time).

FIG. 6(2): The pipette is lowered, and it is detected that an eluate 303L at the tip end of the pipette tip 205 has come into contact with the liquid surface (from lowering of the pipette to detection of the liquid surface). After the detection, aspiration of the eluate is started (start of aspiration (at an aspiration speed of v1)). The amount of liquid to be recovered is preset, and counting of the aspiration amount of the eluate is started from the start of the aspiration (start of counting of the aspiration amount). The counting of the aspiration amount may be defined, for example, by an amount of movement of the plunger. In that case, the plunger is generally driven by a stepping motor, and drive pulses of the stepping motor may be set in accordance with the aspiration amount.

While continuing the aspiration of the eluate, the pipette tip 205 is continuously lowered. At this time, the lowering speed is determined according to the aspiration speed and the shape of the container, and is set to be equal to or greater than the speed at which the liquid surface lowers by the aspiration. By lowering the pipette tip 205 in accordance with the lowering of the liquid surface due to the aspiration in this manner, it is possible to prevent the tip from being exposed to air due to the lowering of the liquid surface and erroneously aspirating the air.

FIG. 6(3): Thereafter, it is detected that the tip end of the pipette tip 205 comes into contact with the upper surface of the magnetic beads 303B that have been magnetically collected and deposited at the bottom of the small container 303 by the bottom magnet 401. At the timing of the detection, the lowering of the pipette is stopped (from detection of the beads to stop of the lowering of the pipette).

At this point, the tip end of the pipette tip 205 is in contact with the upper surface of the magnetic beads 303B, and at that position, the plunger operation speed is reduced to lower the aspiration speed (here, the aspiration speed is denoted as v2 (v1 > v2)). Then, the aspiration operation is continued until the entire recovery amount of the eluate set in advance has been aspirated. The aspiration speed at this time is set such that the aspiration force is lower than the magnetic force of the bottom magnet. In other words, the setting of the aspiration force is made so that the aspiration force estimated from the material and the shape of the pipette tip 205, the plunger diameter, and the like is lower than the magnetic force estimated from the type and the size of the bottom magnet and the physical properties (magnetic permeability, particle size, and the like) of the magnetic beads. By continuing the aspiration under these aspiration conditions, it becomes possible to recover the eluate contained between the magnetic beads at a high recovery rate without aspirating the magnetic beads.

FIG. 6(4): When aspiration of a predetermined amount has been completed, the operation of the plunger is stopped to stop the aspiration (from completion of the aspiration of the predetermined amount to stop of the aspiration). Then, the pipette is moved, and the recovered eluate is discharged into another container. When moving the pipette, a small amount of magnetic beads may have been aspirated into the tip end of the pipette tip 205. Therefore, it is preferable to attract the magnetic beads within the pipette tip 205 by the magnetic force of the bottom magnet 401 and remove the magnetic beads from the inside of the pipette tip 205. Taking the movement time of the magnetic beads into consideration, it is preferable to wait for a predetermined period of time after stopping the aspiration (waiting for a predetermined period of time), and thereafter move the pipette (pipette raising). It should be noted that, in this step, the liquid near the bottom surface of the container is aspirated into the pipette along the surfaces of the beads.

In the steps described above, the detection of the contact between the tip end of the pipette tip 205 and the surface of the eluate, and the detection of the contact with the upper surface of the magnetic beads 303B, can be performed by detecting changes in the aspiration pressure of the pipette using the pressure sensor provided in the pipette.

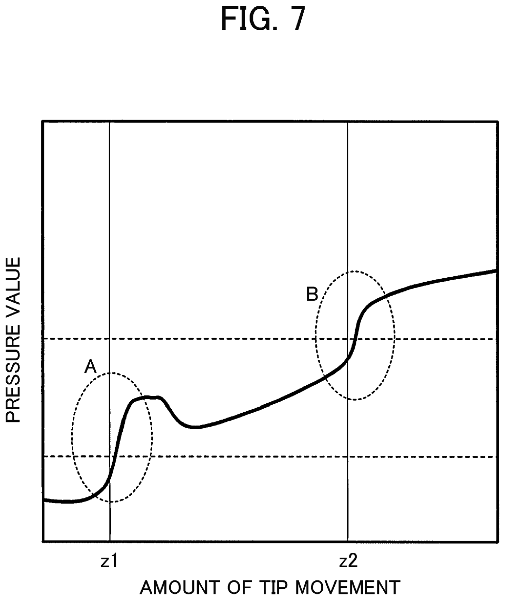

FIG. 7 is a diagram showing changes in the pressure measured by the pressure sensor provided in the pipette during the recovery of the eluate. When the pipette tip 205 is lowered from the initial position and the tip end thereof comes into contact with the liquid surface of the eluate 303L (the amount of tip movement at this time is denoted as z1), the eluate 303L is aspirated, and the pressure changes (portion A in FIG. 7). Furthermore, when the tip end of the pipette tip 205 comes into contact with the upper surface of the magnetically collected and deposited magnetic beads 303B (the lowering amount at this time is denoted as z2), the magnetic beads 303B are aspirated, and the pressure changes (portion B in FIG. 7). By detecting these pressure changes, it is possible to detect a contact between the pipette tip 205 and the liquid surface of the eluate 303L, and a contact between the pipette tip 205 and the upper surface of the magnetic beads 303B.

When the pressure is detected as shown in FIG. 7, if a small amount of magnetic beads 303B enters the pipette tip 205, the magnetic beads may be attracted again toward the bottom of the container by the bottom magnet 401 and discharged from the pipette tip 205.

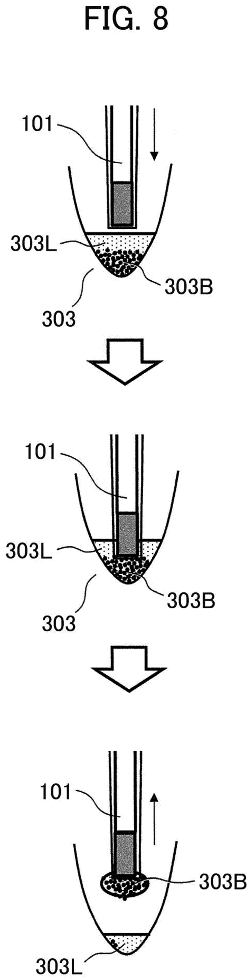

FIG. 8 is a diagram illustrating how loss of an eluate to be recovered occurs in a conventional biological sample processing device. When removing the magnetic beads from the eluate, as shown in FIG. 8, the magnetic rod 101 is placed into the eluate, and the magnetic beads 303B are attracted to the magnet at the tip end of the magnetic rod 101, and the magnetic rod 101 is then pulled out in that state. At this time, the magnetic beads are recovered with the eluate adsorbed thereon, which results in a large-volume loss of the recovered eluate. According to the present embodiment, such a loss can be suppressed.

### <First embodiment: Summary>

In the biological sample processing device 1 according to the first embodiment, the eluate 303L is aspirated by the pipette while the magnetic beads 303B are magnetically attracted by the bottom magnet 401, and when the tip end of the pipette tip 205 comes into contact with the magnetic beads 303B, lowering of the pipette is stopped and the aspiration force is reduced. Accordingly, during the recovery of the eluate, liquid adsorbed to the magnetic beads 303B can also be recovered, and thus it becomes possible to achieve a high recovery rate of the nucleic acid eluate, which is the final product.

### Second Embodiment

A biological sample processing device 1 according to a second embodiment of the present invention has substantially the same configuration as that of the first embodiment and performs similar operations. Therefore, in the following description, explanations of configurations common to both embodiments will be omitted, and the differences between the two embodiments will be mainly described.

In the second embodiment, similarly to the first embodiment, the aspiration is carried out while the surface of the magnetic beads 303B is brought into contact with the tip end of the pipette tip 205 during recovery of the eluate. However, unlike the first embodiment, the position of the surface of the magnetic beads 303B at this time is determined using a preset value. However, due to variations in the amount of the beads and variations in the container installation position, an error may occur between the preset value and the actual position of the upper surface of the magnetic beads. Due to this error, erroneous aspiration of beads or clogging of the tip may occur. The second embodiment aims at solving this problem.

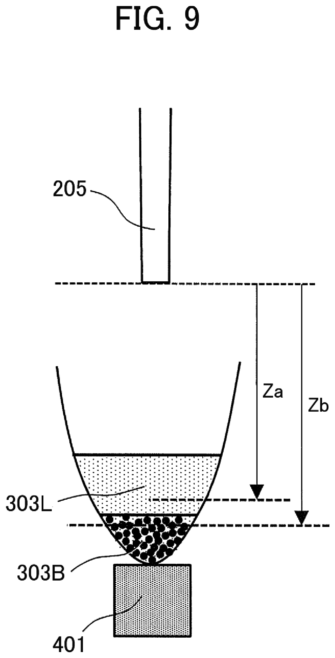

FIG. 9 is a side view illustrating set positions used in an eluate recovery operation according to the second embodiment. In the second embodiment, two values shown in FIG. 9, namely a first set position (Za) and a second set position (Zb), are set. The first set position (Za) is a position at which the tip does not come into contact with the surface of the magnetic beads 303B even when various variations occur, and is a position between the liquid surface of the eluate and the upper surface of the magnetically collected and deposited magnetic beads 303B. The second set position (Zb) is a position at which the magnetic beads 303B and the pipette tip 205 come into contact with each other.

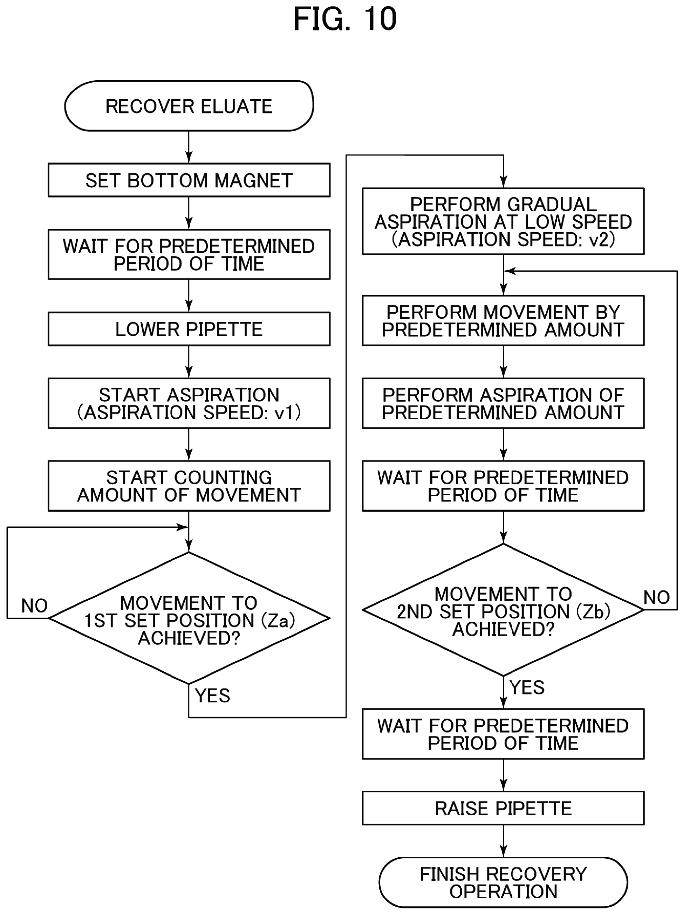

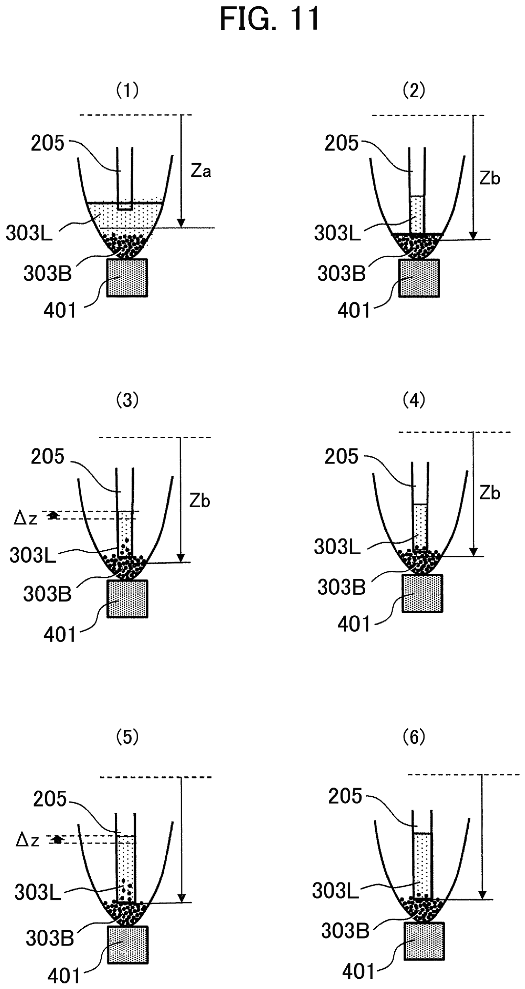

FIG. 10 is a flowchart of the eluate recovery operation after an elution step according to the second embodiment. FIG. 11 is a diagram illustrating an eluate aspiration operation in the eluate recovery operation. Hereinafter, the eluate recovery operation according to the second embodiment will be described with reference to these drawings.

Immediately after the completion of the elution step, the magnetic beads are floating and dispersed in the eluate. Therefore, before starting aspiration of the eluate, the bottom magnet 401 is brought into contact with the bottom of the small container 303, and the device waits for a predetermined period of time. As a result, the magnetic beads 303B are magnetically collected at the bottom of the small container 303 (from setting of the bottom magnet to waiting for a predetermined period of time).

FIG. 11(1): Thereafter, the pipette is lowered, and aspiration by the pipette is started (from lowering of the pipette to start of aspiration (at an aspiration speed of v1)), and aspiration is performed until the first set position (Za) is reached (from start of counting of an amount of movement to determination of whether the movement to the first set position (Za) has been achieved). At this time, the lowering speed and the aspiration speed are determined according to the shape of the container, and are set such that the lowering speed of the pipette is equal to or greater than the speed at which the liquid surface lowers by the aspiration. By lowering the pipette tip 205 in accordance with the lowering of the liquid surface due to the aspiration in this manner, it is possible to prevent the tip from being exposed to air due to the lowering of the liquid surface and erroneously aspirating the air.

FIGS. 11(2) to 11(5): After the pipette has been lowered to the first set position (Za), stepwise aspiration and movement are performed (from performing gradual aspiration at a low speed (at an aspiration of v2) to determination of whether the second set position (Zb) has been achieved). Specifically, the aspiration speed of the pipette is reduced (the aspiration speed is referred to herein as v2), and the aspiration of the predetermined amount, the lowering, and the waiting for the predetermined period of time are repeated until the pipette tip 205 reaches the second set position (Zb). As the waiting time, a period of time sufficient and necessary for the beads to return to their original positions by the magnetic force when the magnetic beads are mixed into the pipette is set.

FIG. 11(6): After the pipette tip 205 comes into contact with the magnetic beads 303B, when a predetermined amount of the eluate is aspirated, a portion of the magnetic beads is aspirated in a state of being mixed. In the aspirated magnetic beads, the magnetic beads in the pipette tip 205 are attracted by the bottom magnet 401 during the waiting time, and the magnetic beads inside the pipette tip 205 are discharged from the pipette tip 205 (waiting for a predetermined period of time). After the magnetic beads are discharged, the pipette is raised (raising of the pipette).

As described above, during the recovery of the eluate, aspiration is carried out while the surface of the magnetic beads and the tip end of the pipette tip are in contact with each other, but the magnetic beads mixed into the pipette tip 205 are discharged from the pipette by the bottom magnet 401. Accordingly, similarly to the first embodiment, liquid adsorbed to the magnetic beads 303B can also be recovered during the recovery of the eluate, and thus it becomes possible to achieve a high recovery rate of the nucleic acid eluate, which is the final product.

In the second embodiment, the first set position Za and the second set position Zb are used instead of using information from the pressure sensor; however, the pressure sensor described in the first embodiment may also be used in combination therewith.

### Third Embodiment

A biological sample processing device 1 according to a third embodiment of the present invention has substantially the same configuration as that of the first embodiment and performs similar operations. Therefore, in the following description, explanations of configurations common to both embodiments will be omitted, and the differences between the two embodiments will be mainly described.

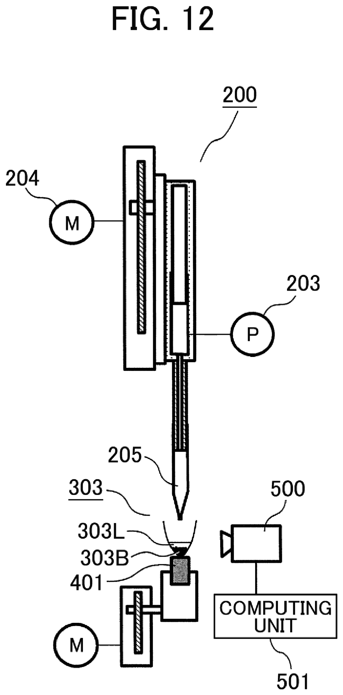

FIG. 12 is a diagram showing a pipette unit 200 of a biological sample processing device 1 according to the third embodiment. In the third embodiment, an image capturing device 500 that captures images of the liquid surface of the eluate and the interface between the magnetic beads and the eluate after magnetic collection is used.

A computing unit 501 acquires in advance a threshold value relating to a color difference between a color of the eluate 303L and a color of the magnetic beads 303B (RGB data or binarized data thereof). Using this threshold value, the computing unit 501 calculates the position in the Z direction of the interface between the two from image data acquired by the image capturing device 500. The computing unit 501 lowers the pipette with respect to the interface position. Alternatively, the computing unit 501 may measure the tip position in the Z direction of the pipette tip 205 from the image data acquired by the image capturing device 500 based on color information or the like thereof. The computing unit 501 may determine a state in which the measured tip end of the pipette tip 205 is in contact with the magnetic bead interface.

The computing unit 501 may detect the liquid surface height of the eluate 303L using the image data acquired by the image capturing device 500. The computing unit 501 lowers the pipette tip 205 in accordance with this height, and starts aspiration from the liquid surface position. Thereafter, the pipette tip 205 is lowered and aspiration is performed (at the aspiration speed of v1) at a predetermined speed until the calculated interface height of the magnetic beads 303B is reached. When the pipette tip 205 is lowered and the tip end of the pipette tip 205 comes into contact with the interface of the magnetic beads 303B, the pipette is stopped. At that position, the aspiration speed is reduced (here, the aspiration speed is referred to as v2, where v1 > v2), and a predetermined amount is aspirated.

Also in the third embodiment, a control for moving the pipette in a stepwise manner as in the second embodiment may be used in combination. Furthermore, the pressure sensor of the first embodiment may also be used in combination.

### Modification of Present Invention

In the above embodiments, as means for detecting the pressure change described with reference to FIG. 7, for example, the computing unit 501 described in the third embodiment may be used. The computing unit 501 may determine a contact by comparing the pressure value or the pressure change rate (time differential value) with a preset threshold value. Alternatively, the computing unit 501 may determine a contact by matching with a standard pressure change pattern. Any other method or a combination thereof may also be employed.

In the above embodiments, the operations described with reference to FIGS. 5 to 6 and the operations described with reference to FIGS. 10 to 11 may be controlled, for example, by the computing unit 501.

The computing unit 501 may be constituted by a hardware such as a circuit device implementing its functions, or may be constituted by a computing device, such as a CPU (Central Processing Unit), executing a software implementing its functions.

In the above embodiments, v1 is, for example, five times, ten times, or more greater than v2, but is not limited thereto, and the effects of the present invention can be achieved as long as at least v1 > v2 is satisfied. However, it can be said that the larger v1 is, the higher the speed at which the liquid is aspirated is.

In the above embodiments, the aspiration force by which the magnetic beads are aspirated by aspiration at v1 may be weaker than the attraction force by which the bottom magnet 401 attracts the magnetic beads. If the aspiration force at v1 is weak, the possibility that the magnetic beads are aspirated into the pipette while the pipette is being lowered decreases, which is advantageous in that respect. However, since the speed of aspirating the liquid also decreases, if importance is placed on the liquid aspiration efficiency, it can be said that the larger v1 is better.

### List of Reference Signs

1: Biological Sample Processing Device
100: Magnetic Rod Unit
101: Magnetic Rod
102: Magnetic Rod Cover
103: Magnetic Rod Drive Motor
104: Magnetic Rod Cover Drive Motor
200: Pipette Unit
201: Plunger
202: Plunger Drive Motor
203: Pressure Sensor
204: Pipette Upward and Downward Movement Motor
205: Pipette Tip
206: Horizontal Movement Motor
300: Container Conveying Unit
301: Large Container
302: Medium Container
303: Small Container
304: Container Holder
305: Container Movement Motor
400: Bottom Magnet Unit
401: Bottom Magnet
500: Image Capturing Device

## Claims

1. A biological sample processing device configured to process a biological sample containing magnetic beads, the device comprising:
a magnet configured to be arranged so as to contact a bottom surface of a container containing the biological sample; and
a pipette configured to aspirate the biological sample in the container or discharge the biological sample into the container,
wherein the pipette performs an aspiration operation in a state in which a tip end of the pipette is in contact with the magnetic beads, and
wherein, after the aspiration of the biological sample, the pipette waits for a period of time sufficient for the magnetic beads in the pipette to be discharged to the outside of the pipette by the magnet.

2. The biological sample processing device according to Claim 1,
wherein the pipette performs an aspiration operation at a first aspiration speed while lowering in a state in which the tip end of the pipette is immersed in the biological sample,
wherein the pipette performs an aspiration operation at a second aspiration speed lower than the first aspiration speed in a state in which the tip end of the pipette is in contact with the magnetic beads, and
wherein, after performing the aspiration operation at the second aspiration speed, the pipette waits for a period of time sufficient for the magnetic beads in the pipette to be discharged to the outside of the pipette by the magnet.

3. The biological sample processing device according to Claim 2, further comprising
a computing unit configured to detect a contact between the tip end of the pipette and the magnetic beads,
wherein, when the tip end of the pipette comes into contact with the magnetic beads, the pipette stops the aspiration operation at the first aspiration speed and stops lowering.

4. The biological sample processing device according to Claim 3,
wherein an aspiration force by which the magnetic beads are aspirated at the second aspiration speed is smaller than an attraction force by which the magnet attracts the magnetic beads.

5. The biological sample processing device according to Claim 1,
wherein an aspiration force by which the pipette aspirates the magnetic beads is smaller than an attraction force by which the magnet attracts the magnetic beads.

6. The biological sample processing device according to claim 3, further comprising
a pressure sensor configured to measure a pressure inside the pipette,
wherein the computing unit detects that the tip end of the pipette has come into contact with the magnetic beads by comparing the pressure measured by the pressure sensor with a threshold value.

7. The biological sample processing device according to claim 2,
wherein the pipette lowers to a first set position below a liquid surface of the biological sample and above an upper surface of the magnetic beads while performing the aspiration operation at the first aspiration speed, and
wherein, after lowering to the first set position, the pipette repeats lowering by a predetermined amount, performing the aspiration operation at the second aspiration speed, and waiting for a predetermined period of time, until reaching a second set position below the upper surface of the magnetic beads.

8. The biological sample processing device according to claim 7,
wherein, after lowering to the second set position, the pipette performs an operation of waiting for a period of time sufficient for the magnetic beads in the pipette to be discharged to the outside of the pipette by the magnet.

9. The biological sample processing device according to claim 3, further comprising
an imaging device configured to image a side surface of the container,
wherein the computing unit detects that the tip end of the pipette has come into contact with the magnetic beads by specifying respective positions of the tip end of the pipette and the magnetic beads based on an image captured by the imaging device.

10. The biological sample processing device according to claim 3, further comprising
an imaging device configured to image a side surface of the container,
wherein the computing unit specifies a liquid surface height of the biological sample based on an image captured by the imaging device, and
wherein the pipette starts the aspiration operation at the first aspiration speed after lowering to the liquid surface height of the biological sample.
